# DEMANDE DE BREVET EUROPEEN

(11) **EP 3 208 256 A1**
(43) Date de publication de la demande: **23.08.2017**
(21) Numéro de dépôt: 17161960.4
(22) Date de dépôt: 21.05.2010
(51) Int. Cl.: C07C 17/25, C07C 21/18

(54) **PROCEDE DE PREPARATION DE COMPOSES FLUORES**

(30) Priorité: 23.07.2009 FR 0955138
(62) Demande divisionnaire de: 10734502.7
(71) Demandeur: Arkema France, 92700 Colombes (FR)
(72) Inventeur: DEVIC, Michel, 69110 SAINTE FOY LES LYON (FR); DOUCET, Nicolas, 57420 CUVRY (FR); WENDLINGER, Laurent, 69510 SOUCIEU EN JARREST (FR); CAVALLINI, Géraldine, SHANGHAI, 201702 (CN); SEDAT, Pierre-Marie, 69210 LENTILLY (FR); AVRIL, Karine, 69006 LYON (FR)
(74) Mandataire: Leca, François Michel

(57) **Abrégé**

L'invention a pour objet un procédé de préparation de fluoropropènes de formule (I) CF₃CF=CHR dans laquelle R représente un atome d'hydrogène ou de fluor à partir d'au moins un composé de formule (Ia) CF₃CF=CFR dans laquelle R a la même signification que dans la formule (I) comprenant les étapes suivantes: (i) hydrogénation d'au moins un composé de formule (Ia) dans un réacteur adiabatique en présence d'un catalyseur avec de l'hydrogène présent en quantité surstochiométrique pour donner un hydrofluoropropane; (ii) condensation partielle du flux issu du réacteur adiabatique de l'étape (i) pour donner une fraction en phase gazeuse, comprenant de l'hydrogène non réagi et une partie d' hydrofluoropropane formé, qui est recyclée à l'étape (i) et une fraction en phase liquide comprenant le reste de l'hydrofluoropropane; (iii) déhydrofluoration d'hydrofluoropropane provenant de la fraction liquide de l'étape (ii)à l'aide d'hydroxyde de potassium dans un milieu réactionnel aqueux contenu dans un réacteur agité pour donner le fluoropropène de formule (I) ; et (iv) purification du fluoropropène obtenu à l'étape (iii).

## Description

L'invention a pour objet un procédé de préparation de composés oléfiniques fluorés.

Les hydrofluorocarbones (HFC) et en particulier les hydrofluorooléfines, telles que le 2,3,3,3-tétrafluoro-1-propène (HFO 1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC (chlorofluorocarbones) et des HCFC(hydrochlorofluorocarbones), qui sont potentiellement dangereux pour la couche d'ozone, les HFOs ne contiennent pas de chlore et donc ne posent pas de problème pour la couche d'ozone.

Le 1,2,3,3,3- pentafluoropropène (HFO-1225ye) est un intermédiaire de synthèse dans la fabrication du 2,3,3,3-tétrafluoro-1-propène (HFO-1234yf).

On connaît plusieurs procédés de fabrication de composés oléfiniques fluorés.

Le document Knunyants et al, Journal of the USSR Academy of Sciences, Chemistry Department, "reactions of fluoro-olefins", report 13., "catalytic hydrogenation of perfluoro-olefins", 1960, décrit de façon distincte diverses réactions chimiques sur des composes fluorés. Ce document décrit l'hydrogénation sensiblement quantitative de l'hexafluoropropène (HFP) sur un catalyseur à base de palladium supporté sur alumine, la température passant de 20°C à 50°C, puis étant maintenue à cette valeur. Ce document décrit la déhydrofluoration du 1,1,1,2,3,3-hexafluoropropane (HFC-236ea) par passage au travers d'une suspension de KOH dans l'éther de dibutyle, pour produire du 1,2,3,3,3-pentafluoropropène-1 (HFO-1225ye) avec un rendement de 60 % seulement. Ce document décrit l'hydrogénation du 1,2,3,3,3-pentafluoropropène-1 (HFO-1225ye) en 1,1,1,2,3-pentafluoropropane (HFC-245eb) sur un catalyseur palladium supporté sur alumine. Au cours de cette hydrogénation il se produit aussi une réaction d'hydrogénolyse, une quantité significative de 1,1,1,2-tetrafluoropropane étant produite. Ce document décrit la déhydrofluoration de 1,1,1,2,3-pentafluoropropane (HFC-245eb) en 2,3,3,3-tétrafluoro-1-propène (HFO-1234yf) par passage dans une suspension de KOH en poudre dans l'éther de dibutyle avec un rendement de 70 % seulement. Ces réactions sont décrites indépendamment les unes des autres, mêmes s'il est indiqué qu'il est possible de les combiner pour synthétiser une gamme de dérivés d'éthylène, propylène et isobutylène contenant des quantités variables de fluor.

Le document US-P-5396000 décrit la préparation de 1,1,1,2,3-pentafluoropropane par déhydrofluoration catalytique de 1,1,1,2,3,3-hexafluoropropane (HFC-236ea) en 1,2,3,3,3-pentafluoropropène-1 (HFO-1225ye), suivi d'une hydrogénation pour produire le composé recherché. La déshydrohalogénation du HFC-236ea en HFO-1225ye est effectuée en phase gazeuse, le produit de la réaction étant, dans un exemple, envoyé directement au réacteur suivant dans lequel a lieu l'hydrogénation du composé HFO-1225ye en composé HFC-245eb. Il est aussi indiqué dans ce document que le composé HFC-236ea peut être obtenu par hydrogénation de hexafluoropropylène (HFP).

Le document US-P-5679875 décrit la préparation de 1,1,1,2,3-pentafluoropropane par déhydrofluoration catalytique de 1,1,1,2,3,3-hexafluoropropane (HFC-236ea) en 1,2,3,3,3-pentafluoropropène-1 (HFO-1225ye), suivi d'une hydrogénation pour produire le composé recherché. Les réactions sont effectuées en phase gaz. Il est aussi indiqué dans ce document que le composé HFC-236ea peut être obtenu par hydrogénation de hexafluoropropylène (HFP).

Le document WO 2008/030440 décrit la préparation du HFO-1234yf à partir du HFO-1225ye en faisant réagir le HFO-1225ye avec de l'hydrogène en présence d'un catalyseur pour donner le HFC-245eb, puis en faisant réagir le HFC-245eb soit avec une solution aqueuse basique en présence d'un catalyseur de transfert de phase et un solvant non-aqueux, non-alcoolique soit en phase gazeuse en présence d'un catalyseur. La réaction d'hydrogénation peut être mise en oeuvre à une température comprise entre 50 et 300°C ou entre 50 et 200°C.

Le document WO 2008/030440 suggère l'emploi d'un réacteur tubulaire pour l'étape d'hydrogénation du HFO-1225ye. Ce document est complètement silencieux sur l'exothermicité de la réaction d'hydrogénation ou la durée de vie du catalyseur.

Le document WO 2008/057794 décrit un procédé de fabrication de composés oléfiniques fluorés ayant de trois à six atomes de carbone et un degré de substitution d'halogène N comprenant (i) la conversion en au moins deux étapes réactionnelles d'une matière première oléfinique fluorée ayant le même nombre de carbone que le composé oléfinique fluoré recherché mais un degré de substitution d'halogène N+1 pour donner un alcane fluoré ayant un degré de substitution d'halogène N+1 et (ii) la conversion de l'alcane fluoré produite en (i) en composés oléfiniques fluorés recherchés.

Selon le document WO 2008/057794, l'utilisation de plusieurs étapes de la conversion (i) pour donner l'alcane fluoré a pour but d'augmenter la conversion et la sélectivité.

Le Tableau 1 de ce document illustre la conversion (i) en au moins quatre étapes avec des températures différentes et de quantités de catalyseur différentes pour chaque étapes.

La conversion (i) en plusieurs étapes du document WO 2008/057794 n'est pas facile à mettre en oeuvre à l'échelle industrielle.

Il existe un besoin d'un procédé de préparation de composés oléfiniques fluorés intégré dont la mise en oeuvre à l'échelle industrielle est plus aisée et/ou fiable tout en permettant d'obtenir des composés oléfiniques fluorés de haute pureté avec de bon rendement.

L'invention fournit donc un procédé de préparation de fluoropropènes de formule (I) : CF₃CF=CHR dans laquelle R représente un atome d'hydrogène ou de fluor à partir d'au moins un composé de formule (Ia) CF₃CF=CFR dans laquelle R a la même signification que dans la formule (I), comprenant les étapes suivantes :
(i)hydrogénation dans un réacteur adiabatique en présence d'un catalyseur d'au moins un composé de formule (Ia) avec de l'hydrogène en quantité surstoechiométrique pour donner au moins un hydrofluoropropane;
(ii)condensation partielle du flux issu du réacteur adiabatique de l'étape (i) pour donner une fraction en phase gaz, comprenant de l'hydrogène non réagi et une partie d' hydrofluoropropane formé à l'étape (i), qui est recyclée à l'étape d'hydrogénation et une fraction en phase liquide comprenant le reste d'hydrofluoropropane formé en (i) ;
(iii)déhydrofluoration de l'hydrofluoropropane provenant de la fraction liquide de l'étape (ii) à l'aide d'hydroxyde de potassium (KOH) dans un milieu réactionnel aqueux contenu dans un réacteur agité pour donner le fluoropropène de formule (I); et
(iv) purification du fluoropropène de formule (I).

De préférence, le catalyseur utilisé à l'étape (i) est un catalyseur de palladium supporté sur alumine, de préférence sur alumine α.

Le procédé selon la présente invention peut comprendre en outre:
- une étape de traitement du sel de potassium co-produit à l'étape de déhydrofluoration (iii)pour régénérer l'hydroxyde de potassium
- une étape de chauffage du flux gazeux recyclé au réacteur adiabatique.

Selon un mode de réalisation de la présente invention, le 1,2,3,3,3-pentafluoropropène est obtenu à partir de l'hexafluoropropène.

Selon un autre mode de réalisation de la présente invention, le 2,3,3,3-tétrafluoro-1-propène est obtenu à partir du 1,2,3,3,3-pentafluoropropène.

Le procédé selon la présente invention peut être mis en oeuvre en discontinu, semi-continu ou continu. Avantageusement, le procédé est mis en oeuvre en continu.

La demanderesse a constaté que le recyclage d'une partie d'hydrofluoropropane formé en (i) permet de contrôler la forte exothermicité de la réaction d'hydrogénation de l'étape (i) et ainsi améliorer la durée de vie du catalyseur. En outre, la condensation partielle de l'étape (ii) permet d'éviter l'entraînement de l'hydrogène dans les étapes ultérieures facilitant ainsi la récupération du fluoropropène de haute pureté avec un bon rendement.

### Etape d'hydrogénation (i)

L'étape d'hydrogénation peut être mise en oeuvre en présence d'un rapport molaire H₂/composé de formule (Ia) compris entre 1,1 et 40, de préférence compris entre 2 et 15.

L'étape d'hydrogénation peut être mise en oeuvre à pression comprise entre 0,5 et 20 bars absolu et de préférence entre 1 et 5 bars absolu.

Comme catalyseurs susceptibles d'être utilisés dans l'étape d'hydrogénation, on peut notamment citer les catalyseurs à base d'un métal du groupe VIII ou rhénium, éventuellement supporté, par exemple sur carbone, carbure de silicium, alumine, fluorure d'aluminium.

Comme métal, on peut utiliser du platine ou du palladium, en particulier du palladium, avantageusement supporté sur carbone ou alumine. On peut aussi associer ce métal avec un autre métal comme l'argent, le cuivre, l'or, le tellure, le zinc, le chrome, le molybdène et le thallium.

Le catalyseur peut être présent sous toute forme appropriée, extrudés, pastilles ou billes.

De préférence, on utilise un catalyseur comprenant entre 0,05 et 10% en poids et avantageusement entre 0,1 et 5% en poids de palladium supporté sur de l'alumine ou carbone.

L'étape d'hydrogénation peut être mise en oeuvre dans des conditions telles que la température à l'entrée du réacteur adiabatique est comprise entre 30 et 200 °C, de préférence entre 40 et 140°C et celle à la sortie du réacteur adiabatique est comprise entre 50 et 250°C, de préférence entre 80 et 160°C.

Le temps de contact (rapport entre le volume de catalyseur et le flux total gazeux dans les conditions normales de température et de pression) est de préférence compris entre 0,2 et 10 secondes et avantageusement entre 1 et 5 secondes.

Cette étape d'hydrogénation peut être mise en oeuvre dans un réacteur adiabatique multiétagé.

L'étape d'hydrogénation du composé de formule (Ia) est sensiblement quantitative.

### Etape de condensation (ii)

Le flux à l'issue de l'étape d'hydrogénation (i) est soumis à une étape de condensation dans des conditions telles que l'hydrogène non réagi n'est pas condensé et qu'une partie d'hydrofluoroalcane formé à l'étape (i) est condensé.

De préférence, l'étape de condensation est mise en oeuvre à une température comprise entre 0 et 50 °C et à une pression comprise entre 0,5 et 20 bar absolu, avantageusement entre 1 et 5 bars absolu.

De préférence, l'étape de condensation est mise en oeuvre dans des conditions telles qu' entre 1 et 30 % de l'hydrofluoroalcane en sortie du réacteur à l'étape (i) est condensé et avantageusement entre 2 et 10 % est condensé.

La fraction non condensée est ensuite recyclée à l'étape d'hydrogénation (i) après un éventuel chauffage.

### Etape de déhydrofluoration (iii)

La fraction condensée comprenant de l'hydrofluoropropane, après vaporisation, est ensuite soumise à une étape de déhydrofluoration à l'aide de l'hydroxyde de potassium présent en quantité, de préférence comprise entre 20 et 75 % en poids et avantageusement comprise entre 55 et 70 % en poids par rapport au poids du mélange eau et KOH du milieu réactionnel aqueux contenu dans le réacteur agité.

Le milieu réactionnel aqueux de l'étape de déhydrofluoration est de préférence maintenu à une température comprise entre 80 et 180 °C, avantageusement comprise entre 125 et 180°C. Une température du milieu réactionnel particulièrement préférée est comprise entre 145 et 165°C.

L' étape de déhydrofluoration peut être mise en oeuvre à une pression de 0,5 à 20 bars mais on préfère travailler à une pression comprise entre 0,5 et 5 bars absolus et plus avantageusement entre 1,1 et 2,5 bars absolus.

Lorsque le composé à hydrogéner est l'hexafluoropropène, l'étape de déhydrofluoration consiste à faire réagir le 1,1,1,2,3,3-hexafluoropropane (HFC-236ea) avec de l'hydroxyde de potassium pour donner le 1,2,3,3,3-pentafluoropropène.

Lorsque le composé à hydrogéner est le 1,2,3,3,3-pentafluoropropène, l'étape de déhydrofluoration consiste à faire réagir le 1,1,1,2,3,-pentafluoropropane (HFC-245eb) avec de l'hydroxyde de potassium pour donner le 2,3,3,3-tetrafluoropropène.

### Etape de traitement pour régénérer l'hydroxyde de potassium

Au cours de l'étape de déhydrofluoration, il se forme du fluorure de potassium.

Le procédé selon la présente invention peut comprendre une étape de traitement au cours de laquelle le fluorure de potassium co-produit à l'étape de déhydrofluoration est mis en contact avec de l'hydroxyde de calcium dans un milieu réactionnel aqueux à une température de préférence comprise entre 50 et 150 °C et avantageusement comprise entre 70 et 120°C et plus avantageusement entre 70 et 100°C.

Cette étape de traitement est de préférence mise en oeuvre en introduisant de l'hydroxyde de calcium dans un réacteur contenant une partie du milieu réactionnel, provenant de l'étape de déhydrofluoration et comprenant du fluorure de potassium, de l'hydroxyde de potassium et de l'eau après éventuelle dilution.

Le fluorure de potassium est de préférence présent entre 4 et 45 % en poids par rapport au milieu réactionnel provenant de l'étape de déhydrofluoration (iii).

Le milieu réactionnel du traitement comprend de préférence entre 4 et 50 % en poids d'hydroxyde de potassium et avantageusement entre 10 et 35 % en poids d'hydroxyde de potassium par rapport au poids total d'hydroxyde de potassium et eau dans le milieu.

L'étape de traitement avec l'hydroxyde de calcium permet de régénérer de l'hydroxyde de potassium qui peut être recyclé à l'étape de déhydrofluoration et d'obtenir du fluorure de calcium de qualité commerciale valorisable après séparation par exemple filtration et décantation.

Du fluorure de calcium de taille moyenne comprise entre 20 et 35 µm (taille moyenne à 50 % en poids de la distribution granulométrique)est obtenu dans les conditions préférées de cette étape de traitement.

L'étape de traitement à l'hydroxyde de calcium peut être mise en oeuvre dans tout type de réacteur connu de l'homme de l'art, par exemple un réacteur agité.

### Etape de purification du fluoropropène de formule (I)

Le flux gazeux à l'issue de l'étape de déhydrofluoration (iii) comprenant le fluoropropène de formule (I), le composé de formule (Ia) non réagi et les sous produits est soumis à une étape de purification pour obtenir des fluoropropènes de haute pureté.

La purification comprend de préférence une première étape de distillation pour séparer les impuretés légères et une deuxième étape de distillation pour séparer le fluoropropène de formule (I) des impuretés lourdes.

En l'absence de l'étape de condensation (ii), de l'hydrogène se retrouve dans les impuretés légères de la première étape de distillation.

Lorsque le produit recherché est le 1,2,3,3,3-pentafluoropropène, les impuretés légères à éliminer comprend le trifluorure de vinyle.

Lorsque le produit recherché est le 2,3,3,3-tetrafluoropropène, les impuretés légères à éliminer comprend le trifluoropropyne.

Lorsque le produit recherché est le 1,2,3,3,3-pentafluoropropène, les impuretés lourdes à éliminer comprennent le 1,1,1,2,3,3 hexafluoropropane (HFC-236ea) qui peut être recyclé à l'étape (iii).

Lorsque le produit recherché est le 2,3,3,3-tetrafluoropropène, les impuretés lourdes à éliminer comprennent le 1,1,1,2,3 pentafluoropropane (HFC-245eb) qui peut être recyclé à l'étape (iii).

On rappellera que:
- le taux de conversion est le % du produit de départ ayant réagi (nombre mole de produit départ ayant réagi/nombre mole produit départ introduit) ;
- la sélectivité en produit recherché est le ratio nombre de mole de produit recherché formé/nombre mole produit départ ayant réagi ;

### EXEMPLE

L'exemple suivant illustre l'invention sans la limiter.

Synthèse du HFO-1225ye par hydrogénation de l'HFP en HFC-236ea puis déhydrofluoration du HFC-236ea en HFO-1225ye.

### Hydrogénation de HFP en HFC-236ea

On utilise un réacteur tubulaire en inox de diamètre interne 2,1 cm et longueur 120 cm contenant 479 g, soit 330 cm³ de catalyseur sous forme d'un lit fixe. Le catalyseur contient 0,2 % en poids de palladium supporté sur alumine α.

Pendant la durée de la réaction, on injecte en continu environ 0,71 mol/h d'hydrogène et 0,7 mol/h de hexafluoropropène. La pression est de 2 bar absolue. Le ratio molaire hydrogène/HFP à l'entrée du réacteur est de 6. La température à l'entrée de réacteur est de 43,2 ° C et la température maximale atteinte au cours de la réaction est de 122,3 ° C. Le temps de contact suivant la définition donnée ci-dessus est de 2,8 s.

On obtient une conversion de 100% en HFP avec une sélectivité en HFC-236ea supérieure à 99%.

Le flux en sortie de réacteur est refroidi à 17,6°C et partiellement condensé. La phase gaz est recyclée au réacteur après avoir été préalablement préchauffée. La phase liquide, représentant 5% du HFC-236ea présent en sortie du réacteur, est composée à plus de 99% de HFC-236ea. Elle est vaporisée avant d'alimenter le réacteur de dehydrofluoration.

### Déshydrofluoration du HFC-236ea en HFO-1225ye

On utilise un réacteur agité de 3 litres en inox de diamètre interne 1,5 cm et contenant 2,6 litres d'une solution de potasse à 60 % environ en poids.

Pendant la durée de la réaction, on injecte en continu environ 0,7 mol/h de HFC-236ea gaz et 6,4 mol/h environ d'une solution liquide de potasse à 70 % environ en poids de KOH (soit 2,7 mol/h de KOH pur). La pression est de 1,1 bar absolue.

La température à l'entrée de réacteur est de 160 °C.

On obtient une conversion de 98% en HFC-236ea avec une sélectivité en HFO-1225ye (E+Z) supérieure à 99%. La pureté du HFO-1225ye (E+Z) est supérieure à 97%.
Mode de réalisation 1 : Procédé de préparation de fluoropropènes de formule (I) : CF₃CF=CHR dans laquelle R représente un atome d'hydrogène ou de fluor à partir d'au moins un composé de formule (Ia) CF₃CF=CFR dans laquelle R a la même signification que dans la formule (I), comprenant les étapes suivantes :
   (i)hydrogénation dans un réacteur adiabatique en présence d'un catalyseur d'au moins un composé de formule (Ia) avec de l'hydrogène en quantité surstoechiométrique pour donner au moins un hydrofluoropropane;
   (ii)condensation partielle du flux issu du réacteur adiabatique de l'étape (i) pour donner une fraction en phase gaz, comprenant de l'hydrogène non réagi et une partie d' hydrofluoropropane formé à l'étape (i), qui est recyclée à l'étape d'hydrogénation et une fraction en phase liquide comprenant le reste d'hydrofluoropropane formé en (i) ;
   (iii)déhydrofluoration de l'hydrofluoropropane provenant de la fraction liquide de l'étape (ii) à l'aide d'hydroxyde de potassium (KOH) dans un milieu réactionnel aqueux contenu dans un réacteur agité pour donner le fluoropropène de formule (I); et
   (iv) purification du fluoropropène de formule (I).
Mode de réalisation 2 : Procédé selon le mode de réalisation 1, dans lequel le rapport molaire hydrogène / composé de formule (Ia) est compris entre 1,1 et 40, de préférence compris entre 2 et 15.
Mode de réalisation 3 : Procédé selon mode de réalisation 1 ou 2, dans lequel la température à l'entrée du réacteur adiabatique de l'étape (i) est comprise entre 30 et 200°C, de préférence entre 40 et 140 °C et celle à la sortie comprise entre 50 et 250°C, de préférence entre 80 et 160°C.
Mode de réalisation 4 : Procédé selon l'un quelconque des modes de réalisation 1 à 3 caractérisé en ce que la pression est comprise entre 0,5 et 20 bars absolu, de préférence entre 1 et 5 bars absolu.
Mode de réalisation 5 : Procédé selon l'un quelconque des modes de réalisation 1 à 4 caractérisé en ce que l'étape de condensation (ii) est mise en oeuvre à une température comprise entre 0 et 50 °C et une pression comprise entre 0,5 et 20 bar absolu, de préférence entre 1 et 5 bar absolu.
Mode de réalisation 6 : Procédé selon l'un quelconque des modes de réalisation 1 à 5 caractérisé en ce que l'hydroxyde de potassium représente entre 20 et 75 %, de préférence entre 55 et 70% en poids du mélange eau-hydroxyde de potassium de l'étape (iii).
Mode de réalisation 7 : Procédé selon l'un quelconque des modes de réalisation 1 à 6 caractérisé en ce que le milieu réactionnel de l'étape de déhydrofluoration (iii) est mis en oeuvre à une température comprise entre 125 et 180°C, de préférence comprise entre 145 et 165°C.
Mode de réalisation 8 : Procédé selon l'un quelconque des modes de réalisation 1 à 7 caractérisé en ce qu'il comprend une étape de traitement au cours de laquelle le fluorure de potassium, co-produit dans l'étape de déhydrofluoration réagit avec de l'hydroxyde de calcium, pour régénérer de l'hydroxyde de potassium.
Mode de réalisation 9 : Procédé selon le mode de réalisation 8 caractérisé en ce que l'hydroxyde de potassium représente entre 4 et 50 % en poids par rapport au poids total d'hydroxyde de potassium et eau du milieu réactionnel aqueux de l'étape de traitement.
Mode de réalisation 10 : Procédé selon le mode de réalisation 8 ou 9 caractérisé en ce la température de traitement est comprise entre 50 et 150°C, de préférence entre 70 et 120°C.
Mode de réalisation 11 : Procédé selon l'un quelconque des modes de réalisation 1 à 10 caractérisé en ce que la purification de l'étape (iv) comprend une première distillation pour éliminer les impuretés légères et une deuxième distillation pour éliminer les impuretés lourdes. Mode de réalisation 12 : Procédé selon l'un quelconque des modes de réalisation 1 à 11 caractérisé en ce qu'il comprend l'hydrogénation de l'hexafluoropropène pour donner du 1,1,1,2,3,3-hexafluoropropane qui est ensuite déhydrofluoré pour donner le 1,2,3,3,3-pentafluoropropène.
Mode de réalisation 13 : Procédé selon l'un quelconque des modes de réalisation 1 à 11 caractérisé en ce qu'il comprend l'hydrogénation du 1,2,3,3,3-pentafluoropropène pour donner du 1,2,3,3,3-pentafluoropropane qui est ensuite déhydrofluoré pour donner le 2,3,3,3-tetrafluoropropène.

## Revendications

1. Procédé de préparation de fluoropropènes de formule (I) : CF₃CF=CHR dans laquelle R représente un atome d'hydrogène ou de fluor à partir d'au moins un composé de formule (Ia) CF₃CF=CFR dans laquelle R a la même signification que dans la formule (I), comprenant les étapes suivantes :
(i)hydrogénation dans un réacteur adiabatique en présence d'un catalyseur d'au moins un composé de formule (Ia) avec de l'hydrogène en quantité surstoechiométrique pour donner au moins un hydrofluoropropane;
(ii)condensation partielle du flux issu du réacteur adiabatique de l'étape (i) pour donner une fraction en phase gaz, comprenant de l'hydrogène non réagi et une partie d' hydrofluoropropane formé à l'étape (i), qui est recyclée à l'étape d'hydrogénation et une fraction en phase liquide comprenant le reste d'hydrofluoropropane formé en (i) ;
(iii)déhydrofluoration de l'hydrofluoropropane provenant de la fraction liquide de l'étape (ii) à l'aide d'hydroxyde de potassium (KOH) dans un milieu réactionnel aqueux contenu dans un réacteur agité pour donner le fluoropropène de formule (I); et
(iv) purification du fluoropropène de formule (I), **caractérisé en ce que** le catalyseur utilisé à l'étape (i) est un catalyseur de palladium supporté sur alumine, de préférence sur alumine α.

2. Procédé selon la revendication 1, dans lequel le rapport molaire hydrogène / composé de formule (Ia) est compris entre 1,1 et 40, de préférence compris entre 2 et 15.

3. Procédé selon la revendication 1 ou 2, dans lequel la température à l'entrée du réacteur adiabatique de l'étape (i) est comprise entre 30 et 200°C, de préférence entre 40 et 140 °C et celle à la sortie comprise entre 50 et 250°C, de préférence entre 80 et 160°C.

4. Procédé selon l'une quelconque des revendications précédentes **caractérisé en ce que** la pression est comprise entre 0,5 et 20 bars absolu, de préférence entre 1 et 5 bars absolu.

5. Procédé selon l'une quelconque des revendications précédentes **caractérisé en ce que** l'étape de condensation (ii) est mise en oeuvre à une température comprise entre 0 et 50 °C et une pression comprise entre 0,5 et 20 bar absolu, de préférence entre 1 et 5 bar absolu.

6. Procédé selon l'une quelconque des revendications précédentes **caractérisé en ce que** l'hydroxyde de potassium représente entre 20 et 75 %, de préférence entre 55 et 70% en poids du mélange eau-hydroxyde de potassium de l'étape (iii).

7. Procédé selon l'une quelconque des revendications précédentes **caractérisé en ce que** le milieu réactionnel de l'étape de déhydrofluoration (iii) est mis en oeuvre à une température comprise entre 125 et 180°C, de préférence comprise entre 145 et 165°C.

8. Procédé selon l'une quelconque des revendications précédentes **caractérisé en ce qu'**il comprend une étape de traitement au cours de laquelle le fluorure de potassium, co-produit dans l'étape de déhydrofluoration réagit avec de l'hydroxyde de calcium, pour régénérer de l'hydroxyde de potassium.

9. Procédé selon la revendication 8 **caractérisé en ce que** l'hydroxyde de potassium représente entre 4 et 50 % en poids par rapport au poids total d'hydroxyde de potassium et eau du milieu réactionnel aqueux de l'étape de traitement.

10. Procédé selon la revendication 8 ou 9 caractérisé en ce la température de traitement est comprise entre 50 et 150°C, de préférence entre 70 et 120°C.

11. Procédé selon l'une quelconque des revendications précédentes **caractérisé en ce que** la purification de l'étape (iv) comprend une première distillation pour éliminer les impuretés légères et une deuxième distillation pour éliminer les impuretés lourdes.

12. Procédé selon l'une quelconque des revendications précédentes **caractérisé en ce qu'**il comprend l'hydrogénation de l'hexafluoropropène pour donner du 1,1,1,2,3,3-hexafluoropropane qui est ensuite déhydrofluoré pour donner le 1,2,3,3,3-pentafluoropropène.

13. Procédé selon l'une quelconque des revendications 1 à 11 **caractérisé en ce qu'**il comprend l'hydrogénation du 1,2,3,3,3-pentafluoropropène pour donner du 1,2,3,3,3-pentafluoropropane qui est ensuite déhydrofluoré pour donner le 2,3,3,3-tetrafluoropropène.
